(19) Europäisches Patentamt
European Patent Office
Office européen des brevets

(11) Publication number: 0 624 588 A1

# (12) EUROPEAN PATENT APPLICATION

(21) Application number: 94107134.2

(22) Date of filing: 06.05.94

(51) Int. Cl.5: C07D 495/22, A61K 31/55, //(C07D495/22,333:00,249:00, 243:00,221:00)

(30) Priority: 07.05.93 ES 9300982

(43) Date of publication of application: 17.11.94 Bulletin 94/46

(84) Designated Contracting States: AT BE CH DE DK ES FR GB GR IE IT LI LU MC NL PT SE

(71) Applicant: J. URIACH & CIA. S.A.
Degà Bahi, 59-67
E-08026 Barcelona (ES)

(72) Inventor: Carceller, Elena
Aragon 117
ES-08015-Barcelona (ES)
Inventor: Recasens, Nuria
Olesa 75-77
ES-08027-Barcelona (ES)
Inventor: Almansa, Carmen
Independència 333
ES-08026 Barcelona (ES)
Inventor: Bartroli, Javier
Cardenal Vives i Tuto 55
ES-08034 Barcelona (ES)

(74) Representative: Zumstein, Fritz, Dr. et al
Patentanwälte,
Dr. F. Zumstein,
Dipl.-Ing. F. Klingseisen,
Bräuhausstrasse 4
D-80331 München (DE)

## (54) New quinoline derivatives with pharmacological activity.

(57) The present invention relates to new quinoline derivatives of formula **I**

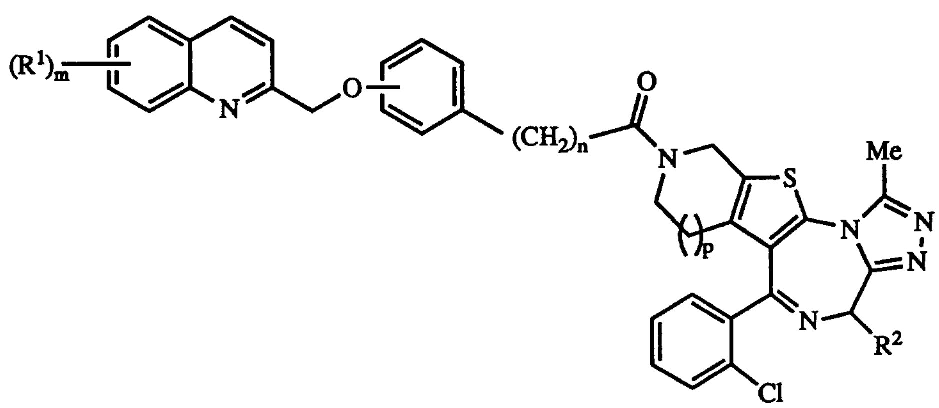

I

wherein: $R^1$ represents fluoro or chloro; $R^2$ represents hydrogen or $C_{1-4}$ alkyl; m is 0, 1 or 2; n is 0 or 1; and p is 0 or 1. These compounds are PAF antagonists and/or 5-lipoxygenase inhibitors.

EP 0 624 588 A1

Field of the invention.

The present invention relates to new quinoline derivatives which are potent platelet activating factor (PAF) antagonists and/or 5-lipoxygenase enzyme inhibitors. The invention also relates to a process for their preparation, to pharmaceutical compositions containing them and to their use in the treatment of diseases in which PAF and/or 5-lipoxygenase are involved.

Description of the prior art.

The platelet activating factor (PAF) or (1-O-alkyl-2-acetyl-*sn*-glyceryl-3-phosphorylcholine), also called acetyl glyceryl ether phosphorylcholine (AGEPC) or PAF-acether, is a natural phospholipid synthesized by different cells (basophiles, macrophages, neutrophiles, platelets) and tissues (heart, lung and kidney) of the organism.

PAF was described for the first time as a potent platelet aggregating agent. Later on it was demonstrated to have other biological activities *in vivo*, such as peripheral vasodilatation, increase of the vascular permeability, induction of bronchoconstriction and hyperreactivity of the respiratory tract. PAF also produces immediate hypotension followed by pulmonary and renal hypertension in rats, guinea pigs, rabbits and dogs, and it has been rated as the most potent ulcerogenic agent described until now.

Consequently, PAF is a mediator that is implicated in a large set of pathological processes such as asthma, septic shock, transplant rejection, thrombosis, ulceration, inflammation and renal diseases.

On the other hand, other cellular mediators such as the leukotrienes have been implicated as important mediators in several inflammmatory diseases such as asthma, rheumatoid arthritis, psoriasis and inflammatory bowel disease. The leukotrienes are the resulting products of the oxidation of arachidonic acid through the action of the enzyme 5-lipoxygenase. It has been postulated that compounds that act as inhibitors of this enzyme thereby preventing the synthesis of leukotrienes could offer great promise as therapeutic agents in the treatment of those disorders.

In light of this, compounds that exhibit a dual activity as PAF antagonists and 5-lipoxygenase inhibitors could be extremely useful therapeutic agents in the treatment of complex pathologies such as asthma, allergic disorders and other inflammatory diseases.

The closest prior art from the structural point of view is believed to be the compounds disclosed in patent application EP 367110, which relates to diazepines with PAF antagonist activity, different from the compounds of the present invention. The present invention describes new potent PAF antagonists structurally related to those described therein, where the nature of the substituent on the nitrogen atom has been substantially modified. Furthermore, unlike the products disclosed therein, the compounds of the present invention also possess 5-lipoxygenase inhibitor activity. Thus, their ability to affect two different pathways to inflammatory disorders makes them extremely useful as medicinal agents.

Description of the invention.

The present invention relates to new quinoline derivatives of general formula **I**

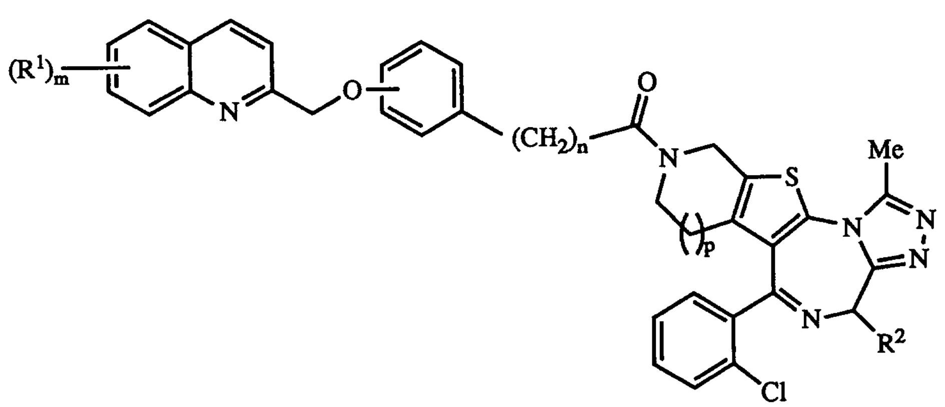

**I**

wherein:

$R^1$ represents fluoro or chloro;

$R^2$ represents hydrogen or $C_{1-4}$ alkyl;
m is 0,1 or 2;
n is 0 or 1;
p is 0 or 1;
and the salts and solvates thereof.

The invention also provides a pharmaceutical composition which comprises an effective amount of a compound of formula **I** or a pharmaceutically acceptable salt or solvate thereof and a pharmaceutically acceptable excipient.

The invention further provides the use of a compound of formula **I** or a pharmaceutically acceptable salt or solvate thereof in the manufacture of a medicament for the treatment or prevention of diseases in which PAF and/or 5-lipoxygenase are involved. Preferred is the use for the treatment or prevention of diseases related with allergy and inflammation such as asthma, rhinitis, dermatitis, urticaria, arthritis and psoriasis; inflammatory bowel disease; and ischemia and shock states such as septic shock, anaphylactic shock, hemorrhagic shock and myocardial ischemia.

The invention still further provides a process for preparing a compound of formula **I** which comprises reacting an acid of general formula **II** or a reactive derivative thereof, such as the acid chloride,

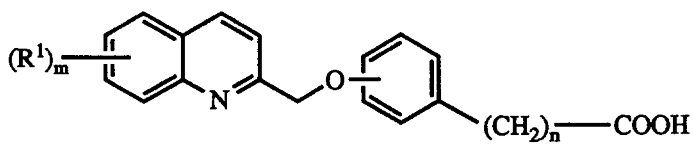

**II**

wherein $R^1$, m and n have the previously defined meaning, with a compound of formula **III**

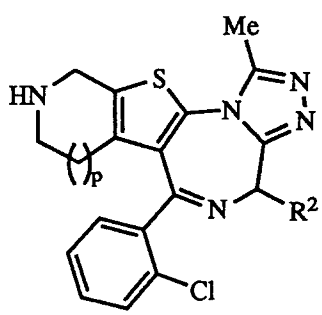

**III**

wherein $R^2$ and p have the previously defined meaning;

and optionally, reacting a compound of formula **I** with an acid to give the corresponding acid addition salt.

Compounds of formula **I** wherein $R^2$ represents $C_{1-4}$ alkyl have one aymmetric center, which gives rise to a pair of enantiomers. The present invention covers both the individual enantiomers as well as their mixtures.

In the above definitions, a $C_{1-4}$ alkyl group means a linear or branched alkyl chain containing from 1 to 4 carbons atoms. It includes methyl, ethyl, propyl, isopropyl, butyl, isobutyl, sec-butyl and tert-butyl, of which methyl and ethyl are preferred and methyl is more preferred.

In the compounds of the present invention, the 2-quinolylmethoxy radical can be in the 2-, 3- or 4-position of the benzene ring, but is preferably in the 3- or 4-position.

In the compounds of the present invention, $R_1$ represents fluoro or chloro. The substituent(s) $R^1$ may be in the 5-, 6- 7- or 8-position of the quinoline ring, but are preferably in the 6- and/or 7-positions of the quinoline ring. Thus, the following substitution patterns are preferred: 6-fluoro, 6-chloro, 7-fluoro, 7-chloro, 6,7-difluoro and 6,7-dichloro.

In the compounds of the present invention, $R_2$ is hydrogen or $C_{1-4}$ alkyl, but preferably is hydrogen or methyl.

Preferred embodiments of the present invention are those compounds of formula **I** wherein the 2-quinolylmethoxy radical is in the 3- or 4-position of the benzene ring, and $R^1$, $R^2$, m, n and p have the previously defined meaning.

More preferred embodiments of the present invention are those compounds of formula **I** wherein the 2-quinolylmethoxy radical is in the 3- or 4-position of the benzene ring, $R_2$ is hydrogen or methyl, and $R^1$, m, n and p have the previously defined meaning.

The formulae of some specific compounds are represented below, together with the number corresponding to the example in which their preparation is described:

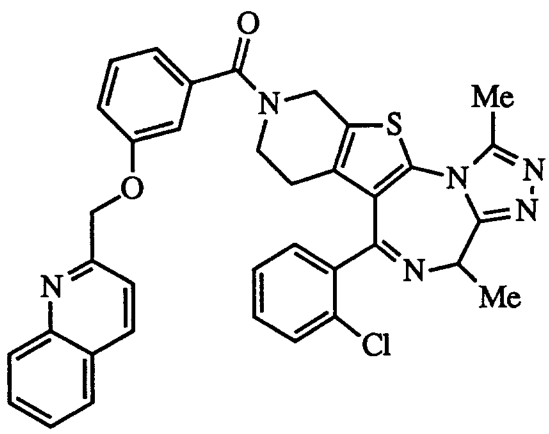

**1**

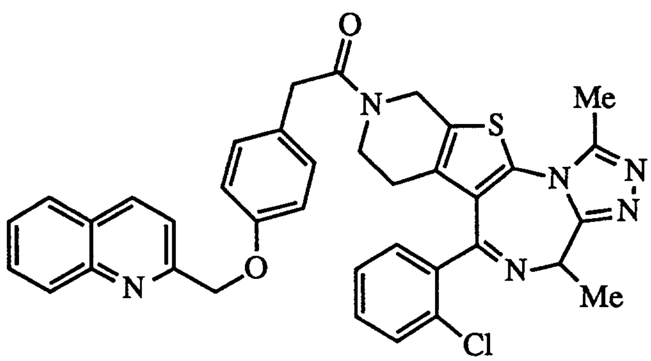

**2**

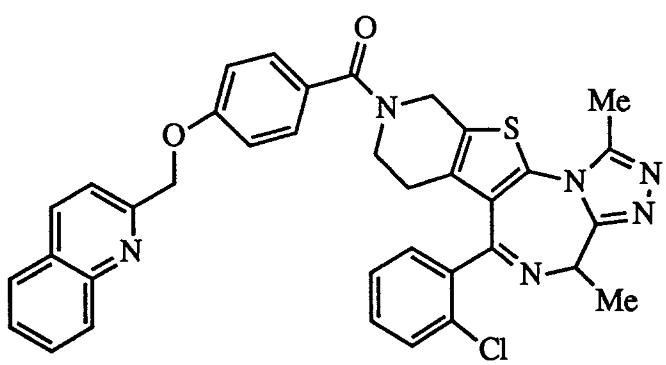

**3**

The compounds of formula **I** contain basic nitrogen atoms and, consequently, they can form salts with acids, which are also included in the present invention. There is no limitation on the nature of these salts, provided that, when used for therapeutic purposes, they are pharmaceutically acceptable, which, as is well-known in the art, means that they do not have reduced activity (or unacceptable reduced activity) or increased toxicity (or unacceptable increased toxicity) compared with the free compounds. Examples of these salts include: salts with an inorganic acid such as hydrochloric acid, hydrobromic acid, hydriodic acid, nitric acid, perchloric acid, sulfuric acid or phosphoric acid; and salts with an organic acid, such as methanesulfonic add, trifluoromethanesulfonic acid, ethanesulfonic acid, benzenesulfonic acid, *p*-toluenesulfonic acid, fumaric acid, oxalic acid, maleic acid, citric acid; and other mineral and carboxylic acids well

known to those skilled in the art. The salts are prepared by reacting the free base with a sufficient amount of the desired acid to produce a salt in the conventional manner. Free bases and their salts differ in certain physical properties, such as solubility, but they are equivalent for the purposes of the invention.

The compounds of the present invention can exist in unsolvated as well as solvated forms, including hydrated forms. In general, the solvated forms, with pharmaceutically acceptable solvents such as water, ethanol and the like, are equivalent to the unsolvated forms for the purposes of the invention.

Some compounds of the present invention can exist as optical isomers because of the existence of an asymmetric carbon in their skeleton. These stereoisomers and the mixtures thereof are all included in the present invention. The optical isomers can be resolved using any of the conventional techniques of optical resolution to give optically pure isomers. Such a resolution can be performed in any chiral synthetic intermediate as well as in the products of general formula **I**. The optically pure isomers can also be individually obtained using enantiospecific synthesis. As stated above, the present invention covers the individual isomers as well as their mixtures (e.g. racemic mixtures), whether as obtained by synthesis or by physically mixing them up.

The invention also provides a process for the preparation of the compounds of formula **I**, as shown in the following scheme:

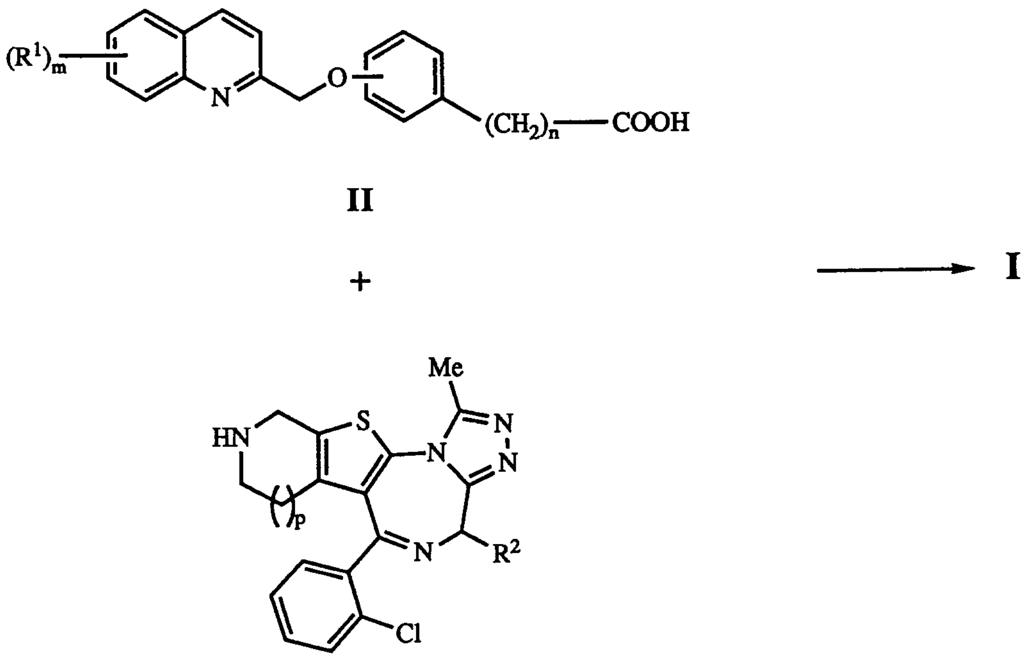

**II**

+

**I**

**III**

Wherein $R^1$, $R^2$, m, n and p have the previously defined meaning.

Compounds of formula **I** are prepared by a dehydration procedure between amines of formula **III** and carboxylic acids of general formula **II**. This process can be carried out by using any conventional reaction of amide bond formation, such as the following processes:

a) By reacting an acid of general formula **II** with 1-hydroxybenzotriazole and dicyclohexylcarbodiimide to form *in situ* an activated ester, and subsequently reacting said ester with an amine of formula **III**. As one skilled in the art recognizes, a wide variety of activated esters can be used in place of that formed by 1-hydroxybenzotriazole. In addition, diimides other than dicyclohexylcarbodiimide can also be employed. The reaction is carried out in a reaction-inert solvent such as dioxane, tetrahydrofuran, acetonitrile, chloroform or N,N-dimethylformamide. The reaction is performed at a temperature ranging from 0 to 60 °C during a period of time from 6 to 24 hours.

b) By reacting amine **III** with an acid chloride or anhydride derived from an acid of general formula **II** in the presence of a proton scavenger amine, such as pyridine or triethylamine, in a suitable solvent such as dichloromethane or chloroform, or the same proton scavenger amine can be used as solvent. The reaction is carried out at a temperature between 0 °C and that of the boiling point of the solvent, during a period of time from 30 min to 24 hours. The compounds thus obtained can be purified by standard

methods such as flash chromatography or crystallization.

The compounds of formula **I** may be transformed into their corresponding acid addition salts following standard procedures, for example by treatment with an acid such as hydrochloric acid, sulfuric acid, nitric acid, oxalic add or methanesulfonic acid.

Amines of formula **III** can be prepared according to the procedures described in EP 367110 and JP 91264589.

Acids of general formula **II** can be obtained by reaction of a 2-chloromethylquinoline derivative hydrochloride of formula **V** with an ester of formula **IV** (wherein R means methyl or ethyl) in the presence of a base such as potassium carbonate in a suitable solvent such as dimethylformamide, at a temperature between room temperature and 80 °C during a period of time from 1 to 24 h; followed by hydrolisis of the intermediate ester thus obtained by treatment with potassium carbonate in a suitable solvent such as methanol-water mixtures, at the temperature of the boiling point of the solvent and during a period of time from 30 min to 12 h.

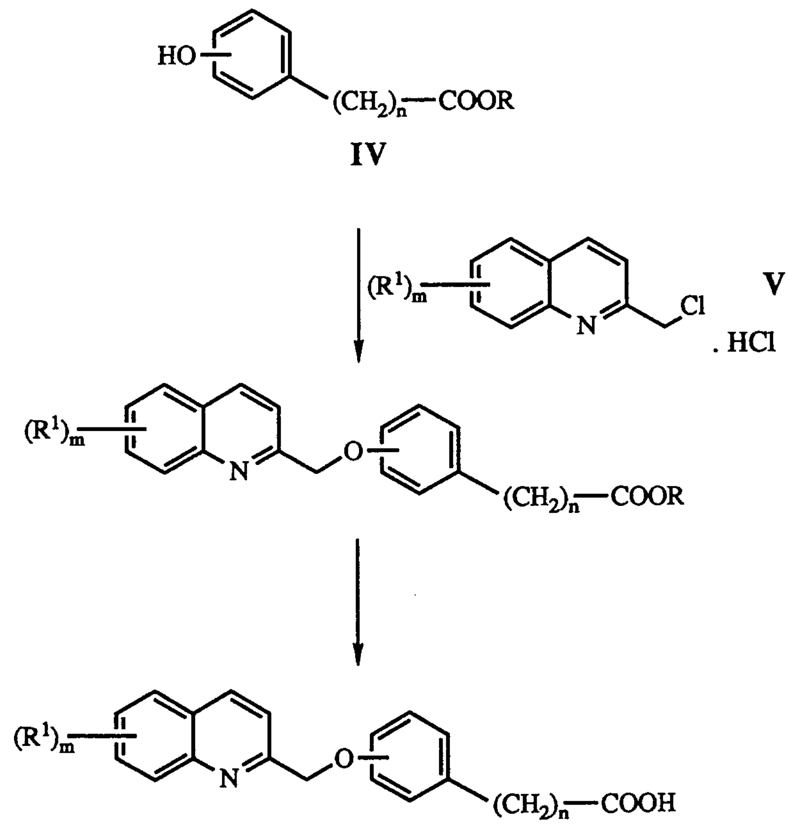

IV

V

. HCl

II

These reactions are all *per se* known ones and can be carried out in accordance with known conditions.

Compounds of formulae **IV** and **V** are either commercially available, or widely described in the literature or can be prepared by methods similar to those described, starting from commercially available products.

The compounds of the present invention possess the capacity to both antagonize PAF and inhibit 5-lipoxygenase enzyme. Therefore, they are useful in the treatment of diseases where PAF and/or 5-lipoxygenase are involved. Being potent PAF antagonists, they are useful as preventive and therapeutic drugs for the treatment of circulatory diseases caused by PAF, such as thrombosis, cerebral apoplexy (e.g. cerebral hemorrhage, cerebral thrombosis), myocardial infarction, angina pectoris, thrombotic phlebitis, trombocytopenic purpura; nephritis (e.g. glomerular nephritis), diabetic nephrosis, pancreatitis; shock states (e.g. septic shock observed after severe infection or postoperatively, intravascular agglutination syndrome caused by endotoxin, anaphylactic shock, hemorrhagic shock, myocardial ischemia); gastrointestinal tract diseases where PAF is involved (e.g. gastric ulcer, inflammatory bowel disease); asthma and other diseases related to allergy and inflammation (e.g. dermatitis, urticaria, arthritis, psoriasis); pneumonia; rejection due to increased PAF production after implantation of organs; and postoperative organodysfunctions (e.g. in heart,

liver and kidney). Being 5-lipoxygenase enzyme inhibitors and, therefore, inhibitors of leukotriene biosynthesis, they are useful as preventive and therapeutic agents for the treatment of diseases such as asthma, allergy- and inflammation-related disorders (e.g. rhinitis, dermatitis, urticaria, eczema, psoriasis), rheumatoid arthritis, gout and inflammatory bowel disease. Having a dual activity as PAF antagonists and 5-lipoxygenase inhibitors, they are particularly useful for the treatment or prevention of complex pathologies such as asthma and other diseases related to allergy and inflammation (e.g. dermatitis, urticaria, rhinitis, arthritis, psoriasis), inflammatory bowel disease, and ischemia and shock states (e.g. septic shock observed after severe infection or postoperatively, intravascular agglutination syndrome caused by endotoxin, anaphylactic shock, hemorrhagic shock, myocardial ischemia) where several mediators are involved, such as PAF and leukotrienes.

The following pharmacological tests explain the activity of the compounds of the present invention in more detail.

**PHARMACOLOGICAL TEST 1**

Inhibition of platelet aggregation induced by PAF.

Blood is obtained by cardiac puncture of male New Zealand albino rabbits (b.w. 2-2.5 Kg) and coagulation is prevented by adding 1 part of 3.16% sodium citrate dihydrate in 9 parts of blood. Platelet rich plasma (PRP) is prepared by centrifuging the blood at 250xg for 10 min. at 4°C and then it is diluted with platelet poor plasma (PPP) obtained by further centrifuging at 3000xg for 10 min. The platelet count is adjusted to $3x10^5/mm^3$. Platelet aggregation induced by PAF ($C_{18}$, prepared in our laboratory) (15 nM) is determined by the Born nephelometric technique (*J. Physiol.*, **1962**, 162, 67) using an aggregometer Chrono-log 500. The activities of the inhibitors are expressed as the $IC_{50}$ value, that is to say the concentration of drug needed to inhibit platelet aggregation by 50%. The results are shown in table I below.

TABLE I

| Compound No. | $IC_{50}$ (μM) |
|---|---|
| 1 | 0.032 |
| 2 | 0.016 |

The activity of the compounds of formula **I** as 5-lipoxygenase enzyme inhibitors was tested as follows:

**PHARMACOLOGICAL TEST 2**

Inhibition of $LTB_4$ production by human granulocytes.

Human promyelocytes (HL-60 cells) are grown in a RPMI 1640 medium suplemented with 20% heat-inactivated fetal bovine serum, 50 U/mL penicillin and 50 μg/mL streptomycin under an atmosphere of 5% $CO_2$ at 37°C. Cells are exposed to 1.3% DMSO for 5 days in order to differentiate them into mature granulocytes and then are washed and resuspended in Dubelcco's phosphate-buffered saline at $10^6$ cells/mL. HL-60 ($10^6$ cells/mL) are incubated for 15 min at 37°C in the presence or absence (vehicle only) of test compound. Cells are then stimulated by calcium ionophore A23187 (5 x $10^{-6}$ M) for 15 min. $LTB_4$ secreted into the external medium is measured by EIA (enzymo immunoassay) using a commercially available $LTB_4$-EIA kit. Results are expressed as the percent inhibition of $LTB_4$ production as compared to controls.

| Compound No. | % inh. of $LTB_4$ production (at 5 μM) |
|---|---|
| 1 | 72 |

According to the activity of the compounds disclosed, the present invention further provides pharmaceutical compositions that comprise a compound of the present invention together with an excipient and

optionally other auxiliary agents, if necessary. The compounds of the present invention can be administered in different pharmaceutical preparations, the precise nature of which will depend, as it is well known, upon the chosen route of administration and the nature of the pathology to be treated.

Thus, solid compositions according to the present invention for oral administration include compressed tablets, dispersible powders, granules and capsules. In tablets, the active component is admixed with at least one inert diluent such as lactose, starch, mannitol, microcrystalline cellulose or calcium phosphate; granulating and disintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated tablets can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides. Soft gelatin capsules are possible wherein the active ingredient is mixed with water or an oily medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, sodium alginate, polyvinylpirrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods. The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also administered in the form of suppositories or enemas (which include aqueous or oily solutions as well as suspensions and emulsions) for rectal administration of the drug, or as creams, ointments, pastes, lotions, gels, sprays, foams, aerosols, solutions, suspensions or powders for topical use. Such compositions are prepared following conventional procedures, well known to those skilled in the art.

The dosage and frequency of dose may vary depending upon symptoms, age and body weight of the patient, as well as upon the route of administration. In general, the compounds of the invention may be administered orally or parenterally to human patients in a daily dose from 5 to 5000 mg for an adult, preferably a dosage from 25 to 1000 mg, which may be administered either as a single dose or as divided doses. A preferred dosage for human patients is from 0.1 to 50 mg/kg of body weight, more preferably from 0.5 to 10 mg/kg of body weight. However, in particular cases, at the discretion of the attending physician, doses outside the broader range may be required.

The compositions for topical administration will contain 0.5-10% by weight of a compound of formula **I**.

Following are some representative preparations for tablets, capsules, syrups, aerosols, injectables and creams. They can be prepared following standard procedures and they are useful in the treatment of PAF- and/or 5-lipoxygenase-mediated conditions.

| **Tablets** | |
|---|---|
| Compound of formula **I** | 100 mg |
| Dibasic calcium phosphate | 125 mg |
| Sodium starch glycolate | 10 mg |
| Talc | 12.5 mg |
| Magnesium stearate | 2.5 mg |
| | 250.0 mg |

| **Hard gelatin capsules** | |
|---|---|
| Compound of formula **I** | 100 mg |
| Lactose | 197 mg |
| Magnesium stearate | 3 mg |
| | 300 mg |

| **Syrup** | |
|---|---|
| Compound of formula **I** | 0.4 g |
| Sucrose | 45 g |
| Flavouring agent | 0.2 g |
| Sweetening agent | 0.1 g |
| Water to | 100 mL |

| **Aerosol** | |
|---|---|
| Compound of formula **I** | 4 g |
| Flavouring agent | 0.2 g |
| Propylene glycol to | 100 ml |
| Suitable propellent to | 1 unit |

| **Injectable preparation** | |
|---|---|
| Compound of formula **I** | 100 mg |
| Benzylic alcohol | 0.05 ml |
| Propylene glycol | 1 ml |
| Water to | 5 ml |

| **Cream** | |
|---|---|
| Compound of formula **I** | 2 g |
| Dimethyl acetamide | 2 g |
| White paraffin | 25 g |
| Stearic alcohol | 22 g |
| Propylene glycol | 12 g |
| Sodium lauryl sulfate | 1.5 g |
| Methylparabene | 0.3 g |
| Purified water | 31.6 g |

The following examples illustrate, but do not limit, the scope of the present invention:

## REFERENCE EXAMPLE 1

### Methyl 4-(2-quinolylmethoxy)phenylacetate

To a mixture of 2-(chloromethyl)quinoline hydrochloride (1 g, 4.6 mmol) and methyl 4-hydroxyphenylacetate (0.83 g, 5 mmol) in anhydrous dimethylformamide (20 mL), was added potassium carbonate (2.33 g) and the mixture was heated at 60 °C for 8 h. Dimethylformamide was removed, and the residue was partitioned between water and chloroform. The organic phase was dried over sodium sulfate and the solvent was removed, to afford 1.68 g of a crude product that was purified by chromatography on silica gel (ethyl acetate-hexane 1:1), to give 1.2 g of the title compound (yield: 84%).
IR (film) $\nu$: 3024, 2945, 1730, 1595, 1503, 1423, 1242, 1217, 1158, 1050, 826 $cm^{-1}$. $^{1}H$ NMR (80MHz, $CDCl_3$) $\delta$ (TMS): 8.18 (s, 1H, Ar), 8.04 (s, 1H, Ar), 7.8-7.4 (m, 4H, Ar), 7.2-6.9 (m, 4H, Ar), 5.34 (s, 2H, $CH_2O$), 3.64 (s, 3H, $CH_3$), 3.53 (s, 2H, $CH_2CO$).

## REFERENCE EXAMPLE 2

### 4-(2-Quinolylmethoxy)phenylacetic acid

To a solution of the compound obtained in reference example 1 (1.2 g, 3.9 mmol) in methanol (25 mL) was added potassium carbonate (1.23 g) dissolved in water (12.5 mL) and the mixture was heated at reflux for 1 h. Next, methanol was removed, more water was added and the resulting solution was extracted with diethyl ether. The aqueous phase was cooled in an ice bath and was acidified with 5N HCl. The solid thus precipitated was filtered and dried to give 0.7 g of the title compound (yield: 61%).
IR (film) $\nu$: 3200-2400, 1699, 1594, 1499, 1286, 1243, 1223, 1139, 1071, 827, 802 $cm^{-1}$; $^{1}H$ NMR (80MHz, $CDCl_3 + CD_3OD$) $\delta$ (TMS): 8.3-6.9 (complex signal, 10H, Ar), 5.36 (s, 2H, $CH_2O$), 3.54 (s, 2H, $CH_2CO$).

## REFERENCE EXAMPLE 3

### Methyl 3-hydroxybenzoate

To a solution of 3-hydroxybenzoic acid (2 g, 14.5 mmol) in methanol (44 mL) was added concentrated $H_2SO_4$ (0.88 mL) and the mixture was heated at reflux for 18 h. Next, the solvent was removed and the residue was diluted with water and extracted with ethyl acetate. The organic phase was dried over anhydrous sodium sulfate and the solvent was removed, to yield 2.2 g of the desired compound (quantitative yield).
IR (film) $\nu$: 3600-3200, 2947, 1694, 1585, 1447, 1433, 1296, 1230, 1103, 997, 755 $cm^{-1}$; $^{1}H$ NMR (80MHz, $CDCl_3$) $\delta$ (TMS): 7.7-7.0 (complex signal, 4H, Ar), 6.14 (broad s., OH), 3.91 (s, 3H, $CH_3$).

## REFERENCE EXAMPLE 4

### Methyl 3-(2-quinolylmethoxy)benzoate

Following the procedure described in reference example 1, but using the compound obtained in reference example 3 instead of methyl 4-hydroxyphenylacetate, the title compound of the example was obtained.
IR (film) $\nu$: 3054, 2944, 1713, 1669, 1593, 1580, 1440, 1287, 1216, 1089 $cm^{-1}$; $^{1}H$ NMR (80MHz, $CDCl_3$) $\delta$ - (TMS): 8.3-7.2 (complex signal, 10H, Ar), 5.41 (s, 2H, $CH_2O$), 3.89 (s, 3H, $CH_3$).

## REFERENCE EXAMPLE 5

### 3-(2-Quinolylmethoxy)benzoic acid

Following the procedure described in reference example 2, but starting from the compound obtained in reference example 4, the title compound was obtained in 49% yield.
mp: 178-182 °C;
IR (film) $\nu$: 3200-2300, 1699, 1591, 1485, 1320, 1285, 1268, 1234, 1218, 1202, 1073, 831, 770, 752 $cm^{-1}$;
$^{1}H$ NMR (80MHz, $CDCl_3 + CD_3OD$) $\delta$ (TMS): 8.4-7.2 (complex signal, 10H, Ar), 5.41 (s, 2H, $CH_2O$).

**REFERENCE EXAMPLE 6**

**Methyl 4-(2-quinolylmethoxy)benzoate**

Following the procedure described in reference example 1, but using methyl 4-hydroxybenzoate instead of methyl 4-hydroxyphenylacetate, a crude product was obtained that was directly used in the next step as obtained.
$^1H$ NMR (80MHz, $CDCl_3$) $\delta$ (TMS): 8.3-6.9 (complex signal, 10H, Ar), 5.43 (s, 2H, $CH_2O$), 3.87 (s, 3H, $CH_3$).

**REFERENCE EXAMPLE 7**

**4-(2-Quinolylmethoxy)benzoic acid**

Following the procedure described in reference example 2, but starting from the compound obtained in reference example 6, the title compound of this example was obtained (yield: 47%).
mp: > 300 °C;
IR (film) $\nu$: 3439, 3058, 1705, 1599, 1502, 1377, 1249, 1171, 1106, 1037, 775 $cm^{-1}$; $^1H$ NMR (80MHz, DMSO-$d_6$) $\delta$ (TMS): 8.40 (d, J= 8.4Hz, 1H, Ar), 8.1-7.6 (m, 7H, Ar), 7.05 (m, 2H, Ar), 5.43 (s, 2H, $CH_2O$).

**EXAMPLE 1**

**6-(2-Chlorophenyl)-1,4-dimethyl-9-[3-(2-quinolylmethoxy)benzoyl]-7,8,9,10-tetrahydro-4H-pyrido[4',3': 4,5]thieno[3,2-f] [1,2,4]triazolo[4,3-a] [1,4]diazepine**

To a mixture of the acid obtained in reference example 5 (0.39 g, 1.4 mmol), 6-(2-chlorophenyl)-1,4-dimethyl-7,8,9,10-tetrahydro-4H-pyrido-[4',3': 4,5]thieno-[3,2-f] [1,2,4]triazolo[4,3-a] [1,4]diazepine (0.5 g, 1.3 mmol) (obtained according to the procedure described in EP 367110) and 1-hydroxybenzotriazole (0.19 g, 1.4 mmol) in anhydrous dimethylformamide (5 mL), was added under an argon atmosphere dicyclohexylcarbodiimide (0.29 g, 1.4 mmol) and the reaction mixture was stirred at room temperature for 18 h. The solvents were removed under vacuum, the resulting residue was stirred with ethyl acetate and the white solid formed was filtered. The organic solution was washed with saturated solution of sodium bicarbonate, with water and finally with brine, dried over sodium sulfate and the solvents were removed, to afford 0.71 g of a residue that was purified by chromatography on silica gel (chloroform: methanol 5%). 290 mg of the title compound of the example was obtained (yield: 35%).
mp: 135-138 °C;
IR (film) $\nu$: 3048, 2926, 1631, 1592, 1528, 1408, 1284 $cm^{-1}$;
$^1H$ NMR (80MHz, $CDCl_3$) $\delta$ (TMS): 8.15 (t, J= 8.0Hz, 2H, Ar), 7.62 (m, 4H, Ar), 7.34 (m, 5H, Ar), 7.02 (m, 3H, Ar), 5.36 (s, 2H, $CH_2O$), 4.67 (m, 2H), 4.28 (q, J= 6.8Hz, 1H, $\underline{CH}CH_3$), 3.78 (m, 1H), 3.39 (m, 1H), 2.66 (s, 3H, $CH_3$ triazole), 2.12 (d, J= 6.7Hz, 3H, $CH\underline{CH_3}$), 1.84 (m, 2H).

**EXAMPLE 2**

**6-(2-Chlorophenyl)-1,4-dimethyl-9-[4-(2-quinolylmethoxy)phenylacetyl]-7,8,9,10-tetrahydro-4H-pyrido[4',3': 4,5]thieno[3,2-f] [1,2,4]triazolo[4,3-a] [1,4]diazepine**

Following the procedure described in example 1, but using the acid obtained in reference example 2, the title compound of the example was obtained (yield: 35%).
mp: 124-127 °C;
IR (film) $\nu$: 2926, 1640, 1594, 1503, 1238 $cm^{-1}$;
$^1H$ NMR (80MHz, $CDCl_3$) $\delta$ (TMS): 8.14 (t, J= 8.0Hz, 2H, Ar), 7.69 (m, 4H, Ar), 7.31 (m, 4H, Ar), 7.00 (q, J= 5.5Hz, 4H, Ar), 5.36 (s, 2H, $CH_2O$), 4.48 (m, 2H), 4.26 (q, J= 6.9Hz, 1H, $\underline{CH}CH_3$), 3.70 (m, 1H), 3.64 (broad s, 2H, $CH_2CO$), 3.32 (m, 1H), 2.64 (s, 3H, $CH_3$ triazole), 2.08 (d, J= 6.9Hz, 3H, $CH\underline{CH_3}$), 1.70 (m, 2H).

## EXAMPLE 3

**6-(2-Chlorophenyl)-1,4-dimethyl-9-[4-(2-quinolylmethoxy)benzoyl]-7,8,9,10-tetrahydro-4H-pyrido[4',3':4,5]thieno[3,2-f] [1,2,4]triazolo[4,3-a] [1,4]diazepine**

Following the procedure described in example 1, but using the acid obtained in reference example 7, the title compound of the example was obtained (yield: 33%).
mp: 140-143 °C;
IR (film) $\nu$: 3051, 2977, 1624, 1599, 1408, 1241 $cm^{-1}$;
$^1H$ NMR (80MHz, $CDCl_3$) $\delta$ (TMS): 8.17 (t, J= 8.0Hz, 2H, Ar), 7.69 (m, 4H, Ar), 7.35 (m, 6H, Ar), 7.02 (d, J= 8.5Hz, 2H, Ar), 5.43 (s, 2H, $CH_2O$), 4.80 (broad q, J= 11.3Hz, 2H), 4.28 (q, J= 7.2Hz, 1H, $\underline{CH}CH_3$), 3.79 (m, 1H), 3.37 (m, 1H), 2.66 (s, 3H, $CH_3$ triazole), 2.12 (d, J= 6.7Hz, 3H, $CH\underline{CH_3}$), 1.85 (m, 2H).

### Claims

1. A compound of formula **I**:

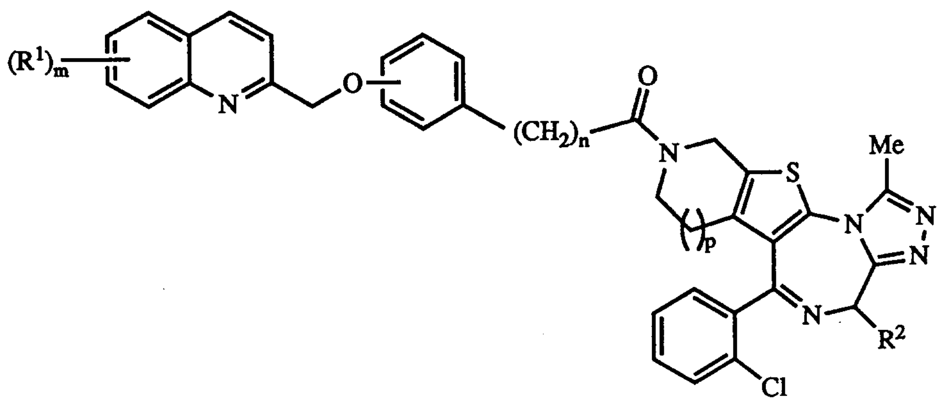

**I**

wherein:
$R^1$ represents fluoro or chloro;
$R^2$ represents hydrogen or $C_{1-4}$ alkyl;
m is 0, 1 or 2;
n is 0 or 1;
p is 0 or 1;
and the salts and solvates thereof.

2. A compound according to claim 1 of formula **I** wherein the 2-quinolylmethoxy radical is in the 3- or 4-position of the benzene ring, and $R^1$, $R^2$, m, n and p have the previously defined meaning.

3. A compound according to claim 1 of formula **I** wherein the 2-quinolylmethoxy radical is in the 3- or 4-position of the benzene ring, $R_2$ is hydrogen or methyl, and $R^1$, m, n and p have the previously defined meaning.

4. 6-(2-Chlorophenyl)-1,4-dimethyl-9-[3-(2-quinolylmethoxy)benzoyl]-7,8,9,10-tetrahydro-4H-pyrido[4',3':4,5]thieno[3,2-f] [1,2,4]triazolo[4,3-a] [1,4]diazepine or a salt or solvate thereof.

5. 6-(2-Chlorophenyl)-1,4-dimethyl-9-[4-(2-quinolylmethoxy)phenylacetyl]-7,8,9,10-tetrahydro-4H-pyrido-[4',3':4,5]thieno[3,2-f][1,2,4]triazolo[4,3-a] [1,4]diazepine or a salt or solvate thereof.

6. 6-(2-Chlorophenyl)-1,4-dimethyl-9-[4-(2-quinolylmethoxy)benzoyl]-7,8,9,10-tetrahydro-4H-pyrido[4',3':4,5]thieno[3,2-f] [1,2,4]triazolo[4,3-a] [1,4]diazepine or a salt or solvate thereof.

**7.** A process for preparing a compound of formula **I** as defined in claim 1 which comprises reacting an acid of general formula **II** or a reactive derivative thereof, such as the acid chloride,

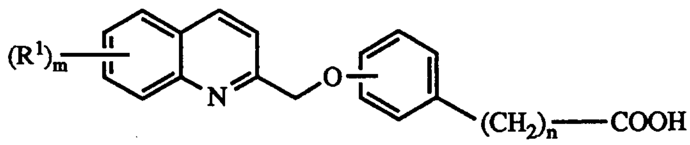

**II**

wherein $R^1$, m and n are as defined in claim 1, with a compound of formula **III**

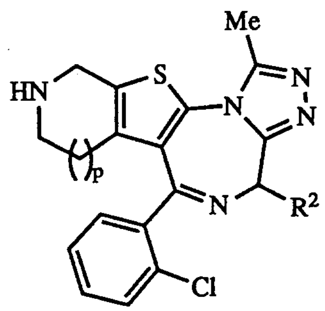

**III**

wherein $R^2$ and p are as defined in claim 1;

and optionally, reacting a compound of formula **I** with an acid to give the corresponding acid addition salt.

**8.** A pharmaceutical composition which comprises an effective amount of a compound of formula **I** as defined in claim 1 or a pharmaceutically acceptable salt or solvate thereof and a pharmaceutically acceptable excipient.

**9.** The use of a compound of formula **I** as defined in claim 1 or a pharmaceutically acceptable salt or solvate thereof in the manufacture of a medicament for the treatment or prevention of diseases in which PAF and/or 5-lipoxygenase are involved.

**10.** The use according to claim 9 wherein the medicament is useful for the treatment or prevention of diseases related with allergy and inflammation such as asthma, rhinitis, dermatitis, urticaria, arthritis and psoriasis.

**11.** The use according to claim 9 wherein the medicament is useful for the treatment or prevention of inflammatory bowel disease.

**12.** The use according to claim 9 wherein the medicament is useful for the treatment or prevention of ischemia and shock states such as septic shock, anaphylactic shock, hemorrhagic shock and myocardial ischemia.

European Patent Office

# EUROPEAN SEARCH REPORT

Application Number
EP 94 10 7134

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A,P | WO-A-94 04537 (CYTOMED, INC.)<br>* page 5, line 10 to page 8, line 17; page 9, lines 13-19; page 21, line26 to page 28, line 13 *<br>--- | 1,8,9 | C07D495/22<br>A61K31/55<br>//(C07D495/22, 333:00,249:00, 243:00,221:00) |
| D,Y | EP-A-0 367 110 (EISAI CO., LTD.)<br>* page 26; pages 30-129, examples; claims 1,18,19 *<br>--- | 1,8,9 | |
| D,Y | PATENT ABSTRACTS OF JAPAN<br>vol. 16, no. 71 (C-0913) 21 February 1992<br>& JP-A-03 264 589 (EISAI CO. LTD.) 25 November 1991<br>--- | 1,8,9 | |
| Y | EP-A-0 503 471 (BOEHRINGER INGELHEIM)<br>* page 8, line 40 to page 10, line 2; page 14, line 54 to page 28, line 50 *<br>--- | 1,8,9 | |
| Y | GB-A-2 231 330 (SCRAS)<br>* pages 20, 23-25; claims 1,6-8 *<br>* page 20; claims *<br>--- | 1,8,9 | **TECHNICAL FIELDS SEARCHED (Int.Cl.5)** |
| Y | GB-A-2 229 723 (SCRAS)<br>* pages 20,21,25; claims 1,6,7 *<br>--- | 1,8,9 | C07D |
| Y | EP-A-0 525 571 (BAYER AG)<br>* page 8, line 55 - page 9, line 25; claims 1,7-10 *<br>--- | 1,8,9 | |
| Y | EP-A-0 499 926 (BAYER AG)<br>* page 10, line 9 - line 39; claims 1,6-10 *<br>--- | 1,8,9 | |
| Y | EP-A-0 414 019 (BAYER AG)<br>* page 8, line 39 - page 9, line 8; claims 1,6-10 *<br>---<br>-/-- | 1,8,9 | |

The present search report has been drawn up for all claims

| Place of search | Date of completion of the search | Examiner |
|---|---|---|
| BERLIN | 25 July 1994 | Hass, C |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number
EP 94 10 7134

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 344 519 (BAYER AG)<br>* page 16, line 4 - line 14; claims 1,7-10 *<br>--- | 1,8,9 | |
| Y | GB-A-2 181 135 (AMERICAN HOME PRODUCTS CORP.)<br>* page 5, line 51 - page 11, line 45; claims 1,25-27 *<br>--- | 1,8,9 | |
| Y | CHEMICAL ABSTRACTS, vol. 106, no. 25, 22 June 1987, Columbus, Ohio, US; abstract no. 207457g, page 44 ;<br>& C.M. TENNANT et al., J. Pharm. Pharmacol. 1987, 39(4), 309-11<br>----- | 1 | |
| | | | TECHNICAL FIELDS SEARCHED (Int.Cl.5) |

The present search report has been drawn up for all claims

| Place of search | Date of completion of the search | Examiner |
|---|---|---|
| BERLIN | 25 July 1994 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document